(19) Europäisches Patentamt
European Patent Office
Office européen des brevets

(11) Numéro de publication: **0 331 556 A2**

(12) # DEMANDE DE BREVET EUROPEEN

(21) Numéro de dépôt: **89400475.3**

(22) Date de dépôt: **21.02.89**

(51) Int. Cl.4: **C 07 C 99/00**
C 07 C 101/72

(30) Priorité: **29.02.88 FR 8802455**

(43) Date de publication de la demande:
**06.09.89 Bulletin 89/36**

(84) Etats contractants désignés:
**BE CH DE ES GB IT LI NL SE**

(71) Demandeur: **MANUFACTURE DE PRODUITS CHIMIQUES PROTEX**
**2, place Joffre**
**F-75007 Paris (FR)**

(72) Inventeur: **Jullien, Jacques André Louis**
**Le Pré Saint Aubin Auzouer en Touraine**
**F-37110 Chateaurenault (FR)**

**Aymard, Alain**
**La Boisselière**
**F-37100 Chateaurenault (FR)**

(74) Mandataire: **Aron, Georges et al**
**Cabinet de Boisse 37, Avenue Franklin D. Roosevelt**
**F-75008 Paris (FR)**

(54) **Nouveau procédé de préparation de l'acide éthylène-diamine-N-N'-bis (ortho-hydroxyphénylacétique) et de dérivés de celui-ci.**

(57) L'invention se rapporte au domaine de la chimie organique.

Elle concerne un procédé de préparation de l'acide éthylène-diamine-N,N'-bis(ortho-hydroxyphénylacétique) et de dérivés de celui-ci, par réaction d'un phénol approprié, d'acide glyoxylique et d'éthylène-diamine dans un solvant polaire aromatique en présence d'une base minérale et d'un catalyseur choisi parmi (i) les oxydes hydroxydes et sels de certains métaux, tels que Zn ou Mg, solubles dans le milieu réactionnel, (ii) les amines tertiaires et (iii) les mélanges de ceux-ci.

Les composés produits sont des agents chélatants.

EP 0 331 556 A2

**Description**

**Nouveau procédé de préparation de l'acide éthylène-diamine-N-N′-bis-(ortho-hydroxyphénylacétique) et de dérivés de celui-ci.**

L'invention concerne un nouveau procédé de préparation de l'acide éthylène-diamine-N-N′-bis(ortho-hydroxyphénylacétique) et de dérivés de celui-ci.

L'acide éthylène-diamine-N-N′-bis(ortho-hydroxyphénylacétique) (en abrégé EDDHA), appelé aussi éthylène-bis(acide α-imino-o-hydroxyphénylacétique, et ses dérivés sont des agents chélatants connus de métaux polyvalents. Les chélates formés avec des métaux polyvalents sont utiles dans des applications agricoles, industrielles et autres. En particulier les chélates formés avec le fer sont utiles pour lutter contre la chlorose de plantes et permettent de cultiver des plantes sujettes à la chlorose dans des sols alcalins qui sont normalement impropres, comme l'enseignent par exemple US-A-2 824 128 et US-A-4 130 582, qui décrivent aussi des procédés de préparation de ces composés et de leurs chélates.

Les procédés connus de préparation peuvent être classés en deux groupes, à savoir le procédé à l'acide cyanhydrique et le procédé au glyoxalate.

Selon le procédé à l'acide cyanhydrique, deux moles d'aldéhyde salicylique sont mises à réagir avec une mole d'éthylène diamine pour donner la base de Schiff correspondante. Celle-ci est mise à réagir avec deux moles d'acide cyanhydrique pour donner un dinitrile, que l'on hydrolyse avec de l'acide chlorhydrique très concentré. On obtient alors le composé désiré.

Le prix relativement élevé de l'aldéhyde salicylique, le nombre d'opérations chimiques élevé, et leurs difficultés opératoires (en particulier l'utilisation d'acide chlorhydrique super concentré à 42% contribuent à rendre cette synthèse difficile et coûteuse.

Selon le procédé au glyoxylate, on fait réagir en une seule étape, deux moles d'un glyoxalate alcalin, avec deux moles de phénol ou de phénol substitué et avec une mole de diamine. Classiquement la réaction est conduite en milieu hydroalcoolique de façon à être en milieu homogène et à une température comprise entre 70 et 100° C correspondant en général à la température d'ébullition du milieu réactionnel et à un pH alcalin de 8 à 10. Malheureusement, quand la synthèse est faite dans un mélange eau et solvant miscible à l'eau tel que méthanol, éthanol ou isopropanol, suivant le mode opératoire décrit dans US-A-2 824 128, l'isomère para, qui ne forme pas un chélate de fer stable sous des conditions alcalines, est obtenu préférentiellement (environ 80%) à l'isomère ortho.

De nombreuses études ont été entreprises pour augmenter le rendement en composé ortho et la plus connue est celle utilisant un grand excès de phénol par rapport à l'éthylène diamine, comme l'enseigne US-A-4 130 582. L'isomère ortho est obtenu préférentiellement à raison de 85 à 95%.

Cette synthèse bien que très séduisante, oblige à utiliser un énorme excès de phénol qui doit être éliminé et nécessite des extractions longues et coûteuses avec des risques considérables de pollution, étant donné que, en raison de la phytotoxicité du phénol, il faut complètement l'éliminer avant de faire la synthèse du chélate métallique.

Il existe donc un besoin pour un procédé de préparation de l'EDDHA et de dérivés de celui-ci, qui soit exempt des inconvénients des procédés antérieurs.

La présente invention vise à satisfaire ce besoin.

La Demanderesse a trouvé d'une façon surprenante, que l'utilisation conjuguée de catalyseurs appropriés et de solvants aromatiques permet d'obtenir un produit réactionnel riche en composé ortho lors de la synthèse des agents chélatants décrits précédemment. Ce procédé présente les avantages de partir de matières premières bon marché et facilement disponibles, de n'avoir recours qu'à un nombre réduit d'étapes chimiques, et d'être peu polluant.

La présente invention concerne donc, un procédé de préparation de l'acide éthylènediamine-N,N′-bis(ortho-hydroxyphénylacétique) et de dérivés de celui-ci répondant à la formule générale suivante :

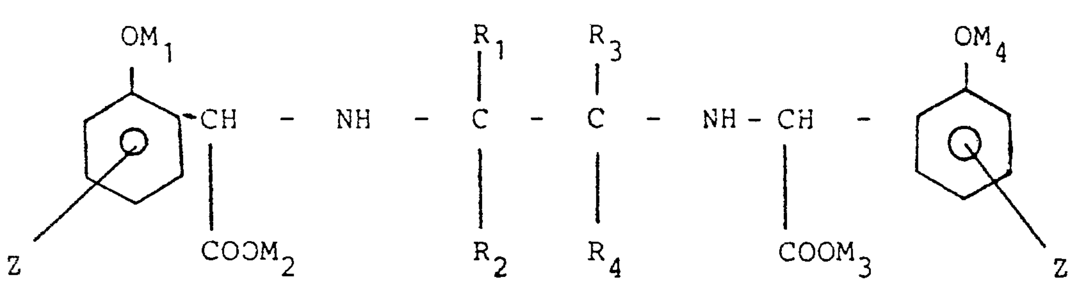

où Z est au moins un substituant choisi parmi l'hydrogène et un substituant non hydrolysable tel qu'un groupe alkyle, alkoxy, carboxyle, acyle, hydroxyle, nitro ou sulfo, $R_1$, $R_2$, $R_3$, $R_4$ sont chacun l'hydrogène ou un groupe alkyle inférieur tel qu'un groupe méthyle, éthyle ou propyle, $M_1$, $M_2$, $M_3$, $M_4$ sont chacun l'hydrogène ou un

métal alcalin, caractérisé en ce qu'on fait réagir un phénol, de l'acide glyoxylique et de l'éthylène diamine, à une température de 60 à 100° C, dans un solvant non polaire aromatique, en présence d'une base minérale et d'un catalyseur choisi dans le groupe formé par (i) les oxydes, hydroxydes et sels de zinc, de magnésium, de manganèse, de cobalt, de fer, de nickel, de cuivre, de baryum, de titane, d'aluminium, de molybdène, de vanadium, d'étain ou de néodyme, et les mélanges de ceux-ci, qui sont solubles dans le milieu réactionnel, et (ii) les amines tertiaires, et (iii) les mélanges de ceux-ci.

La réaction est conduite à une température de 60 à 100° C, de préférence de 85 à 95° C car c'est dans cette gamme de températures que l'on obtient les meilleurs rendements.

Le phénol peut être un phénol, substitué ou non, sur le noyau par au moins un substituant choisi parmi l'hydrogène et les substituants non hydrolysables non susceptibles d'empêcher la réaction de condensation. Des exemples non limitatifs de tels substituants sont les radicaux alkyles et alcoxy et les groupes carboxyle, acyle, hydroxyle, nitro ou sulfo.

Les matières de départ utilisées dans le procédé de l'invention peuvent être employées en quantités sensi blement stoechiométriques, mais on préfère, toutefois, opérer en présence d'un léger excès du phénol afin d'obtenir un meilleur rendement. Par léger excès, on entend une proportion molaire pouvant aller jusqu'à 2,5 fois la quantité stoechiométrique.

La base minérale employée sera habituellement un hydroxyde de métal alcalin, comme la soude ou la potasse, mais d'autres bases minérales pourraient convenir aussi, comme des bicarbonates de métal alcalin. En variante, au lieu d'introduire dans la réaction un hydroxyde ou bicarbonate de métal alcalin, on pourrait partir d'un phénate de métal alcalin ou d'un glyoxalate de métal alcalin préformé.

Comme solvant, on peut utiliser un solvant aromatique non polaire tel que le benzène, le toluène ou le xylène. On préfère le toluène pour des raisons de coût et de toxicité.

Comme catalyseur, on utilise de préférence un oxyde, hydroxyde ou sel (par exemple un acétate, chlorure ou sulfate) de zinc, de magnésium, de manganèse, de cobalt, de fer, de nickel, de cuivre, de baryum, de titane, d'aluminium, de molybdène, de vanadium, d'étain ou de néodyme, et les mélanges de ceux-ci, qui sont solubles dans le milieu réactionnel. On préfère, en particulier, employer un oxyde, hydroxyde ou sel de zinc, de magnésium, de manganèse ou de molybdène, ou un mélange de ceux-ci. Par l'expression "solubles dans le milieu réactionnel", on veut dire que l'oxyde, hydroxyde ou sel utilisé se dissout dans ledit milieu ou réagit avec celui-ci pour former un composé dissous dans ledit milieu. Certains des composés mentionnés, comme le bioxyde de titane, ne sont pas solubles dans le milieu réactionnel et ne conviennent donc pas aux fins de l'invention. La quantité de catalyseur peut aller, à titre indicatif, de 0,001 à 0,05 mole de catalyseur par mole d'acide glyoxylique. En variante, on pourrait utiliser aussi une amine tertiaire, telle que la triéthylamine ou la tributylamine comme catalyseur. Les amines tertiaires sont cependant moins intéressantes que les composés métalliques précités car elles gênent l'élimination du phénol en excès. A la fin de la réaction, qui dure habituellement de 1 à 10 heures, on peut séparer le solvant aromatique du produit désiré en ajoutant une quantité d'eau au mélange réactionnel obtenu, suffisante pour provoquer la formation de deux phases liquides, à savoir une phase aqueuse renfermant le produit désiré et le catalyseur et une phase organique constituée du solvant aromatique et du phénol ou dérivé de phénol inaltéré qui subsiste éventuellement lorsque on a eu recours à l'emploi d'un excès de phénol. Il est alors aisé de séparer ces deux phases par simple décantation. Le catalyseur est diffile à séparer du produit désiré en raison de sa solubilité élevée dans l'eau. Il n'est toutefois pas gênant pour les utilisations habituelles de l'agent chélatant et on peut donc le laisser dans la solution aqueuse obtenue qui peut être utilisée telle quelle pour la formation d'un chélate. Par exemple, on peut former un chélate de fer en ajoutant un sel de fer à cette solution aqueuse et en ajustant le pH entre 6,5 et 7. Comme sel de fer, on peut utiliser, par exemple, les chlorures, sulfates, nitrates et carbonates. On peut ajouter le sel de fer en proportion stoechiométrique, mais on préfère employer un excès de fer compris entre 10 et 20%.

Si désiré, on pourrait extraire et purifier le mélange agent chélatant-catalyseur contenu dans la solution aqueuse en le précipitant de cette solution par acidification à pH 4-5. Une telle acidification a également pour effet de convertir dans le composé obtenu les groupes $M_1$, $M_2$, $M_3$ et $M_4$, qui sont sous forme d'un métal alcalin, en atomes d'hydrogène.

Egalement, il faut noter qu'on pourrait éliminer le solvant et le phénol résiduel éventuel du mélange réactionnel par une opération d'entraînement à la vapeur, plutôt que par la méthode précédemment décrite.

Le procédé de l'invention permet d'obtenir un mélange de composés à configuration ortho et para formé à plus de 60% et parfois à plus de 80% de l'isomère ortho désiré.

Les exemples non limitatifs suivants sont donnés en vue d'illustrer davantage le procédé de l'invention.

EXEMPLE 1

Dans un ballon muni d'une agitation mécanique et d'un réfrigérant, on introduit 600 g de toluène, 94 g (1 mole) de phénol et 28 g (0,5 mole) de potasse. L'eau formée par la formation du phénate est éliminée par distillation azéotropique à 110° C. On refroidit à 30° C et on ajoute 1,1 g (0,006 mole) d'acétate de zinc, 2 g (0,02 mole) de triéthylamine, 74 g (0,5 mole) d'acide glyoxylique à 50% puis 15 g (0,25 mole) d'éthylène-diamine en maintenant la température inférieure à 40° C. Puis, sous forte agitation, on chauffe le mélange réactionnel à 85° C pendant 3 heures, après quoi on refroidit à 20° C. On ajoute 80g d'eau de façon à former un mélange biphasique. On élimine la phase organique par décantation, puis la phase aqueuse est lavée trois fois avec 200 g de chlorure de méthylène. Le rendement en acides éthylène-diamine-N-N′-bis(ortho- et para-hydroxyphénylacétiques) est de 61%, dont 85% de dérivé "ortho", comme déterminé par RMN.

On ajoute 50,8 g de sulfate ferrique heptahydraté à la phase aqueuse lavée. Durant la chélation le pH est maintenu entre 6,5 et 7 par l'addition d'hydroxyde de potassium à 30% en poids. On obtient alors un liquide rouge qui est évaporé à sec. On obtient 109,1 g d'une poudre rouge parfaitement soluble dans l'eau, qui est un chélate de fer de l'EDDHA.

EXEMPLE 2

On répète la synthèse décrite dans l'exemple 1 mais en faisant varier divers paramètres.

Les résultats sont notés dans le tableau suivant :

| Solvants utilisés | Base utilisée | Catalyseur utilisé | Température de réaction | Temps de réaction | Conversion en composé ortho, % | Rendement en composés ortho- et para-hydroxy phénylacétiques par rapport à l'éthylènediamine de départ |
|---|---|---|---|---|---|---|
| Benzène | KOH | ZnO | 65°C | 6 h | 70 | 58 |
| Toluène | NaOH | ZnO | 85°C | 4 h | 60 | 52 |
| Toluène | KOH | ZnO | 90°C | 3 h | 80 | 61 |
| Xylène | NaOH | Acétate de magnésium | 100°C | 3 h | 62 | 52 |
| Toluène | KOH | Acétate de zinc | 80°C | 2 h | 80 | 58 |
| Toluène | KOH | Acétate de magnésium | 85°C | 4 h | 82 | 55 |
| Toluène | KOH | Acétate de zinc | 90°C | 4 h | 82 | 56 |
| Toluène | KOH | Triéthyl amine | 90°C | 4 h | 70 | 54 |
| Toluène | KOH | Acétate de manganèse + Acétate de magnésium (1) | 90°C | 3 h | 66 | 53 |
| Toluène | KOH | oxyde de molybdène + acétate de magné-sium (1) | 85°C | 3 h | 53 | 52 |

(1) les deux constituants du catalyseur sont présents en proportions équimolaires (0,003 mole chacun)

EXEMPLE 3

On répète le mode opératoire de l'exemple 1, si ce n'est que le phénol est remplacé par du paracrésol (108,1 g). On obtient finalement 114,1 g du complexe de fer de l'acide éthylène diamine-N-N'-bis(2-hydroxy 5-méthyl phényl acétique).

EXEMPLE 4

On suit le mode opératoire de l'exemple 1 si ce n'est que le phénol est remplacé par 174g de l'acide paraphénol sulfonique. On obtient finalement 136,2g du complexe de fer de l'acide éthylène diamine-N,N'-bis(2-hydroxy 5-sulfo phényl acétique).

**Revendications**

1. Un procédé de préparation de l'acide éthylène-diamine-N,N'-bis(ortho- hydroxyphénylacétique) et de dérivés de celui-ci répondant à la formule générale suivante :

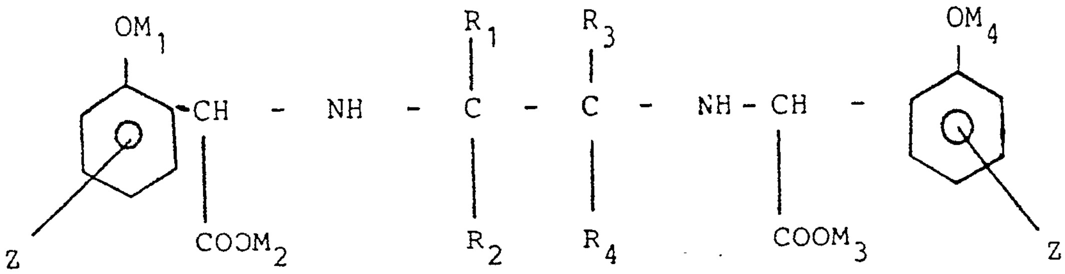

où Z est au moins un substituant choisi parmi l'hydrogène et un substituant non hydrolysable,
$R_1$, $R_2$, $R_3$, $R_4$ sont chacun l'hydrogène ou un groupe alkyle inférieur,
$M_1$, $M_2$, $M_3$, $M_4$ sont chacun l'hydrogène ou un métal alcalin, caractérisé en ce qu'on fait réagir un phénol, de l'acide glyoxylique et de l'éthylène diamine, à une température de 60 à 100° C, dans un solvant non polaire aromatique, en présence d'une base minérale et d'un catalyseur choisi dans le groupe formé par (i) les oxydes, hydroxydes et sels de zinc, de magnésium, de manganèse, de cobalt, de fer, de nickel, de cuivre, de baryum, de titane, d'aluminium, de molybdène, de vanadium, d'étain ou de néodyme, et les mélanges de ceux-ci, qui sont solubles dans le milieu réactionnel et (ii) les amines tertiaires, et (iii) les mélanges de ceux-ci.

2. Procédé selon la revendication 1, caractérisé en ce que, au lieu de partir d'un phénol et d'une base minérale, on remplace ces deux réactifs par un phénate de métal alcalin.

3. Procédé selon la revendication 1 ou 2, caractérisé en ce que le solvant non polaire aromatique est choisi parmi le benzène, le toluène et le xylène.

4. Procédé selon l'une quelconque des revendications 1 à 3, caractérisé en ce que le catalyseur est choisi parmi les oxydes, hydroxydes et sels de zinc, de magnésium, de manganèse et de molybdène, et les mélanges de ceux-ci, qui sont solubles dans le milieu réactionnel.

5. Procédé selon l'une quelconque des revendications 1 à 4, caractérisé en ce que la réaction est conduite à une température de 60-100° C.

6. Procédé selon la revendication 6, caractérisé en ce que la réaction est conduite à une température de 85-95° C.

7. Procédé selon l'une quelconque des revendications 1 à 6, caractérisé en ce qu'on fait réagir des proportions sensiblement stoechiométriques d'éthylène-diamine, d'acide glyoxylique et de base minérale avec une proportion de phénol en excès n'excédant pas environ 2,5 fois la proportion stoechiométrique.

8. Procédé selon l'une quelconque des revendications 1 à 7, caractérisé en ce que, à la fin de réaction, on sépare le solvant aromatique du produit désiré par addition d'une quantité d'eau au mélange réactionnel suffisante pour provoquer la formation de deux phases liquides, à savoir une phase aqueuse renfermant le produit désiré et le catalyseur et une phase organique renfermant le solvant aromatique et le phénol ou dérivé de phénol inaltéré éventuel, puis on sépare la phase aqueuse de la phase organique.

9. Procédé selon l'une quelconque des revendications 1 à 8, caractérisé en ce que Z est choisi parmi H, un groupe alkyle, alkoxy, carboxyle, acyle, hydroxyle, nitro ou sulfo.

10. Procédé selon l'une quelconque des revendications 1 à 8, caractérisé en ce que $R_1$, $R_2$, $R_3$ et $R_4$ sont choisis chacun parmi H, un groupe méthyle, éthyle ou propyle.